# EUROPÄISCHE PATENTSCHRIFT

(11) **EP 1 592 439 B1**
(45) Veröffentlichungstag und Bekanntmachung des Hinweises auf die Patenterteilung: **16.05.2007**
(21) Anmeldenummer: 04710818.8
(22) Anmeldetag: 13.02.2004
(51) Int. Cl.: A61K 38/38

(54) **VERFAHREN ZUR HERSTELLUNG EINER ALBUMINLÖSUNG**
METHOD FOR PRODUCTION OF AN ALBUMIN SOLUTION
PROCEDE DE PRODUCTION D'UNE SOLUTION D'ALBUMINE

(30) Priorität: 13.02.2003 AT 2182003
(43) Veröffentlichungstag der Anmeldung: 09.11.2005
(73) Patentinhaber: Octapharma AG, 8853 Lachen (CH)
(72) Erfinder: GEHRINGER, Werner, A-1160 Wien (AT); POCK, Katharina, A-2125 Streifing (AT); RÖMISCH, Jürgen, A-2440 Gramatneusiedl (AT); SVAE, Tor-Einar, A-2340 Mödling (AT); KANNICHT, Christoph, 10967 Berlin (DE)
(74) Vertreter: Meyers, Hans-Wilhelm
(86) Internationale Anmeldenummer: PCT/EP2004/001397
(87) Internationale Veröffentlichungsnummer: WO 2004/071524

(56) Entgegenhaltungen:
- EP-A- 0 366 946
- DE-A- 19 729 778
- US-A- 5 919 907

## Beschreibung

Die Erfindung betrifft ein Verfahren zur Herstellung. von therapeutisch einsetzbaren virusinaktivierten Albumin.

Albumin ist das im Blutplasma anteilsmäßig am stärksten vertretene Plasmaprotein. Albumin kann viele endogene und exogene Stoffe an sein Molekül binden. Auf dieser Bindungskapazität beruht auch eine seiner Hauptfunktionen: der Transport der an Albumin gebundenen Stoffe.

Aufgrund dieser Bindungskapazität stellt Albumin auch ein wichtiges Depot für eine Vielzahl von Verbindungen dar, wie z.B. langkettige Fettsäuren, Bilirubin, Tryptophan, Thyroxin oder Metallionen. Auch verabreichte pharmazeutische Wirkstoffe, wie Warfarin, Digitoxin oder Naproxen werden an Albumin gebunden und transportiert.

In diesem Zusammenhang ist es jedoch entscheidend zu wissen, dass lediglich der freie Anteil des jeweiligen pharmazeutischen Wirkstoffes, d.h. der nicht an Albumin gebundene, jener ist, der die pharmakologische Wirkung entfaltet. Eine Reduktion des an Albumin gebundenen Teils erhöht den freien Anteil und damit die pharmakologische Aktivität.

Sämtliche der im Handel erhältlichen Albuminpräparate werden mittels einer modifizierten Cohn-Fraktionierung hergestellt, wobei dieses Verfahren in der Regel aus mehreren Fraktionierungsschritten besteht. Die Pasteurisierung (10 Stunden bei 60°C) des Albuminkonzentrates wird als Virusinaktivierungsschritt seit Jahrzehnten angewendet. Zur Vermeidung der Denaturierung von Albumin während dieses Schrittes werden Stabilisatoren zugesetzt. Gemäß Europäischer Pharmacopoeia werden als Stabilisatoren Natriumcaprylat (Natriumoctanoat) oder N-Acetyltryptophan oder eine Kombination von beiden verwendet.

Um Virus inaktivierte Faktor VIII Präparate oder andere Plasmaproteine zu gewinnen, wird das sogenannte SD-Verfahren angewendet, wie z.B. in der EP-A-0 131 740 beschrieben. Auf diese Offenlegungsschrift wird ausdrücklich Bezug genommen.

Der . Anmelderin ist aufgrund eigener Studien bekannt, dass die Bindungskapazität von kommerziell, erhältlichem Albumin im Vergleich zu natürlichem Albumin erheblich beeinträchtigt ist. Dies wird darauf zurückgeführt, dass die bei der Pasteurisierung verwendeten Stabilisatoren vom Albumin gebunden werden und auf diese Weise wichtige Transportstellen besetzen, wodurch die Bindungskapazität abnimmt. Dies bedeutet, dass Patienten, die solche Albuminpräparationen erhalten, bei Verabreichung von pharmazeutischen Wirkstoffen einer beträchtlich erhöhten Konzentration an freiem, d.h. nicht an Albumin gebundenem Wirkstoff ausgesetzt sind, was für den Patienten naturgemäß eine erhöhte Gefahr überschießender pharmakologischer Wirkungen und Nebenwirkungen mit sich bringt.

Die vorliegende Erfindung stellt sich die Aufgabe, eine Albuminpräparation zur Verfügung zu stellen, welche diesen Nachteil nicht aufweist.
Fig. 1 zeigt das UV-Absorptionsverhalten von Albuminen verschiedener Provenienz in Abhängigkeit von der Elutionszeit während der chromatographischen Trennung.
Fig. 2 illustriert das Bindungsverhalten von Albuminen verschiedener Provenienz in Gegenwart von verschiedenen Konzentrationen von Phenylbutazon.
Fig. 3 illustriert das Bindungsverhalten von Albuminen verschiedener Provenienz in Gegenwart von verschiedenen Konzentrationen von Warfarin.

Gelöst wird das Problem durch ein therapeutisch einsetzbares virusinaktiviertes Albumin mit gegenüber durch Pasteurisierung virusinaktiviertem Albumin erhöhter Bindungskapazität für Substanzen. Das erfindungsgemäß herstellbare Albumin weist insbesondere eine um mindestens 10 % erhöhte Bindungskapazität gegenüber durch Pasteurisierung virusinaktiviertem Albumin auf, typischer Weise 20 bis 500 % erhöhte Bindungskapazität, insbesondere 100 % bis 500 % erhöhte Bindungskapazität, auf. In Abhängigkeit von der zu bindenden Substanz sind in Einzelfällen auch noch höhere Werte möglich.

Die Substanzen sind insbesondere solche, die von nativem Albumin gebunden und/oder transportiert werden, wozu insbesondere niedermolekulare Wirkstoffe gehören. Insbesondere sind die niedermolekularen Wirkstoffe organische oder anorganische Substanzen, Nukleinsäuren, Polypeptide, die typischer Weise ein Molekulargewicht von ≤ 10 000 Da aufweisen.

Für die angesprochenen therapeutischen Zwecke kann das erfindungsgemäß herstellbare Albumin in flüssiger Lösung oder in festem Zustand, insbesondere in lyophylisierter Form vorliegen.

Das erfindungsgemäß herstellbare Albumin ist auch erhältlich durch ein Verfahren, welches gekennzeichnet ist durch die Kombination folgender Schritte:
(a) Unterziehen einer ersten wässerigen Albuminlösung einer Behandlung zur Virusinaktivierung nach dem SD-Verfahren durch Kontaktierung mit SD-Reagenzein bei einer Temperatur unter 45° C
(b) Entfernen der SD-Reagenzien durch Ölextraktion und anschließender hydrophober Wechselwirkungschromatographie zumindest im wesentlichen, wobei zur Chromatographie eine hydrophobe Matrix, insbesondere eine Matrix, an welcher gegebenenfalls hydrophobe Gruppen gebunden sein können verwendet wird, mit der Maßgabe, dass diese Gruppen aliphatische Gruppen mit C > 24 sind, und eine zweite Albuminlösung erhalten wird, welche
(c) gegebenenfalls mit einem oder mehreren Stabilisatoren aus der Gruppe Zucker, Aminosäuren und Zuckeralkohole versetzt wird, mit der Maßgabe, dass als Stabilisator kein Indol-Stabilisator und keine C₆-C₁₀-Fettsäure eingesetzt wird, wonach
(d) die gegebenenfalls mit Stabilisator versetzte, zweite Albuminlösung endkonfektioniert und steril filtriert und gegebenenfalls in Endbehältnisse abgefüllt wird.

Der Begriff Indol-Stabilisator soll sämtliche Stabilisatoren umfassen, welche ein Indol-Gerüst aufweisen, also z.B. N-Acetyltryptophan.

Das SD-(=Solvens/Detergens)-Verfahren zur Inaktivierung von Viren ist aus der EP-A - 0 131 740 bekannt. In dieser Druckschrift ist unter anderen Proteinen auch Albumin genannt.

Aus der EP-A - 0 366 946 ist zwar bekannt, dass die SD-Reagenzien mit Pflanzenölen, z.B. Sojaöl, und anschließender hydrophober Wechselwirkungschromatographie entfernt werden können. Das Verfahren gemäß Anspruch 8 ist soweit es sich mit dem Verfahren gemäß EP-A - 0 366 946 überschneidet mithin in einem Aspekt als Analogieverfahren zur Herstellung des erfindungsgemäßen Albumins anzusehen. Zur Chromatographie wird jedoch dort vorzugsweise eine Matrix, z.B. eine Silicamatrix, vorgeschlagen, an welche hydrophobe Seitenketten, und zwar verzweigte oder unverzweigte C₆-C₂₄-Alkylketten gebunden sind.

Es hat sich überraschenderweise gezeigt, dass der Einsatz einer hydrophoben Matrix anstelle einer Matrix, die als hydrophobe Seitenketten beispielsweise C₁₈-Alkylketten trägt, eine höhere Bindungskapazität zur Adsorption von Detergenzien besitzt. Entsprechend brauchen an die erfindungsgemäß verwendete Matrix keine weiteren hydrophoben Gruppen gebunden sein. Ein Verfahren unter Verwendung einer solchen Matrix ist daher auch Gegenstand der Erfindung.

Die Virusinaktivierung wird vorteilhaft bei einer Temperatur im Bereich von 25 bis 40 °C vorgenommen.

Eine bevorzugte Ausführungsform des erfindungsgemäßen Verfahrens besteht darin, dass die Virusinaktivierung während eines Zeitraumes im Bereich von 4 bis 6 Stunden vorgenommen wird.

Als Stabilisator eignet sich sehr gut Glycin.

Zur Ölextraktion eignet sich sehr gut Rizinusöl.

Es hat sich als für den Reinigungseffekt als besonders vorteilhaft erwiesen, wenn als hydrophobe Matrix ein Polystyrol-Divinylbenzol-Polymer oder ein Polymer auf Methacrylat-Basis verwendet wird.

, Die erfindungsgemäß eingesetzten hydrophoben Matrizes können verzweigte oder lineare aliphatische Gruppen mit mehr als 24 C-Atomen tragen.

Abhängig vom verwendeten Ausgangsmaterial kann ein Schritt zur Abreicherung der sogenannten Präkallikrein-Aktivator-Aktivität (PKA) erforderlich sein. PKA ist bekannt dafür, durch Freisetzung der vasoaktiven Substanz Bradykinin aus dem hochmolekularen Kininogen (HMWK) zum Abfall des Blutdruckes nach Administration PKA-haltiger Präparate zu führen.

PKA wird in der Regel während der Pasteurisierung von Proteinpräparaten inaktiviert. Da eine Hitzebehandlung, durch welche die PKA erfahrungsgemäß zumindest partiell inaktiviert wird, aus oben genannten Gründen unvorteilhaft für das verfahrensgemäß hergestellte Albumin ist, kann PKA, falls erforderlich, durch spezielle Maßnahmen entfernt werden. Dazu gehört die Inkubation mit Aktivkohle mit nachfolgender Filtration, bevorzugt mit Tiefenfiltern, oder das direkte Filtrieren durch Aktivkohle-haltige Filter.

Ferner eignen sich Ionenaustauscher, wie Kationen- oder Anionenaustauscher, gut zur Entfernung der PKA. Dies kann durch Kontaktieren der albuminhaltigen Lösung mit der Matrix in Säulen oder dem Fachmann vertrauten Batch-Verfahren erfolgen. Alternativ können Dextransulfat- oder Heparinmatrices zur Reduktion von PKA eingesetzt werden.

In der erhaltenen albuminhaltigen Lösung ist die PKA reduziert, im optimalen Fall nicht mehr nachweisbar. PKA ist nach dem heutigen Stand der Technik identisch mit dem aktivierten (Gerinnungs-) Faktor XII (FXIIa), der aus seiner Proenzymform (FXII) generiert wird. Dies kann an Oberflächen autokatalytisch oder durch enzymatische Einwirkung, beispielsweise des Kallikreins, erfolgen. Entsprechend ist auch eine Abreicherung des FXII (Proenzymes) als Ausgangssubstanz der PKA empfehlenswert, jedoch nicht zwingend erforderlich. Um jedoch einer erneuten Generierung von PKA aus der Proenzymform vorzubeugen, kann diese auch durch Ionenaustauscherchromatographie entfernt werden. Das Abreichern des FXII kann gegebenenfalls durchgeführt werden, um eine langfristige Lagerung des Albumins in flüssigem Zustand zu ermöglichen. Dies ist auch nach Auftauen einer gegebenenfalls in gefrorenem Zustand gelagerten Albuminlösung wichtig. Entsprechend kann die Albuminlösung nach Abfüllung in die Endbehältnisse tiefgefroren werden, jedoch auch in flüssigem oder gefriergetrocknetem Zustand gekühlt und bis zu einer Temperatur von 40°C gelagert werden.

Zur Entfernung des PKA bzw. von PKA-Vorläufersubstanzen kann somit vor oder nach den Schritten (a), (b) oder (c) gegebenenfalls vorhandene Präkallikrein-Aktivator-Aktivität (PKA) in an sich bekannter Weise entfernt werden, wobei die Albuminlösung insbesondere
A) mit Aktivkohle in Kontakt gebracht wird, wonach die Aktivkohle aus der Albuminlösung entfernt wird, oder
B) einer Ionenaustauschchromatographie unterzogen wird.

Der Schritt (A) wird bei einer Albuminkonzentration zwischen 1 und 25 Gew.-%, insbesondere zwischen 5 und 10 Gew.-%, vorgenommen.

Der Schritt (B) wird insbesondere bei einer Albuminkonzentration zwischen 5 und 10 Gew.-% durchgeführt.

Eine weitere Ausführungsform des erfindungsgemäßen Verfahrens besteht darin, dass der Ionenaustauscher ein Anionenaustauscher ist und die Albuminlösung mit Natriumacetat im Bereich von 100 - 150 mMol/l gepuffert ist und der pH im Bereich von 5,0 -6,0, insbesondere <5,5 liegt.

Ferner wird ein Verfahren beschrieben, das dadurch gekennzeichnet ist, dass der Ionenaustauscher ein Kationenaustauscher ist und die Albuminlösung mit Natriumacetat im Bereich von 20 -30 mmol/l gepuffert ist und der pH im Bereich von 4,8 - 6,0, insbesondere im Bereich von 4,8-5,2 liegt.

Das erfindungsgemäße Verfahren ist anwendbar auf Albuminlösungen, gewonnen aus verschiedenen Quellen, z.B. aus Blutplasma oder -serum, aus albuminhaltigen Fraktionen der Plasmafraktionierung, aus Kulturüberstand gewonnenem Albumin nach rekombinanter Herstellung oder transgen hergestelltem Albumin oder aus dem das Albumin enthaltenden Medium, wie Milch.

Eine bevorzugte Ausführungsform der Erfindung wird an Hand des nachfolgenden Beispiels näher beschrieben.

### Beispiel

1000 g einer wässerigen Albuminlösung aus dem Cohn-Verfahren (nach Dia-/Ultrafiltration) mit einem Proteingehalt von etwa 23% werden Triton X-100 und Tri-n-butylphosphat (TNBP) bis zu einer Konzentration von jeweils 1% zugegeben. Anschließend wird die Albuminlösung 4 Stunden bei 30°C gerührt. Zur Entfernung der SD-Reagenzien wird zunächst Rizinusöl unter Rühren bis zu einer Konzentration von 5% zugegeben, während die Lösung auf eine Temperatur im Bereich von 20-25°C gebracht wird. Danach wird das Gemisch 30 Minuten gerührt. Nach dem Rühren wird das 60 Minuten Gemisch ruhen gelassen, wobei sich ein schwere, wässerige Phase und eine leichte Phase ausbilden. Die schwere Phase wird abgetrennt und über ein Filter mit Membranen einer Porengröße von < 1 µm und < 0,45 µm filtriert. Die leichte Phase (Ölphase) enthält das TNBP und wird verworfen.

Die filtrierte Lösung wird zur Abtrennung des Triton X-100 über eine Festphasenextraktionssäule geführt. Als hydrophobe Matrix wird ein Polystyrol-Divinylbenzol-Polymer (Amberchrome CG 161) ohne hydrophobe Seitenketten verwendet. Wasser zur Injektion wird verwendet, um die Säule auszuwaschen, wobei dieser Vorgang durch Messung der UV-Absorption bei 280 nm überwacht wird. Nach Verwendung wird die Säule regeneriert.

Als Stabilisatoren können zugegeben werden: Glyzin, Glutamat, Arginin, Maltose, Sorbit oder Mischungen der Substanzen.

Die erhaltene Lösung wird auf pH=7,0 gebracht und der Proteingehalt auf 200 g/l und der Natriumgehalt auf 80 mMol/l Na⁺ eingestellt. Dann wird die Lösung durch ein Membranfilter mit einer Porengöße von ≤0,2 µm sterilfiltriert.

Die sterilfiltrierte Lösung wird unter aseptischen Bedingungen in sterile, pyrogen-freie PVC-Beutel abgefüllt und etikettiert.

Die etikettierten Beutel werden bei einer Temperatur von <-60°C tiefgefroren, sodass die Temperatur im Inneren der Beutel <-30°C erreicht. Bei dieser Temperatur (≤-30°C) werden die Beutel gelagert.

### Präkallikreinabreicherung

Für den Fall der PKA-Abreicherung kann nach folgenden Varianten vorgegangen werden:
a) Eine Albuminlösung mit einer Proteinkonzentration von 1-25 Gew.-%, insbesondere 5-10 Gew.-% wird mit 3-10 Gew.-%, insbesondere 5 Gew.-% Aktivkohle bei pH = 5 eine Stunde gerührt. Anschließend wird die Aktivkohle ab filtriert.
b) Eine Albuminlösung mit einer Proteinkonzentration von 5-10 Gew.-% wird bei pH 5- 6, insbesondere < 5,5, in einem 100-150 mMol Natriumacetat gepufferten System einer Ionenaustauschchromatographie (DEAE-Sepharose, Q-Sepharose) unterworfen. Durch die hohe Ionenstärke wird im Durchlauf eine PKA freie Albuminlösung gewonnen.
c) Eine Albuminlösung mit einer Proteinkonzentration von 5-10 Gew.-% wird bei pH 5- 6, bevorzugt 4,8-5,2, in einem 20-30 mMol/l Natriumacetat gepufferten System einer Ionenaustauschchromatographie (SP-Toyopearl, CM-Sepharose) unterworfen. Im Durchlauf wird eine PKA-freie Albuminlösung gewonnen.

### Endformulierung

Die erhaltenen Lösungen werden jeweils auf pH = 7,0 gebracht und der Proteingehalt auf 200 g/l und der Natriumgehalt auf 80 mMol/l Na⁺ eingestellt. Dann wird die Lösung durch ein Membranfilter mit einer Porengöße von <0,2 µm sterilfiltriert.

Die sterilfiltrierten Lösungen werden unter aseptischen Bedingungen in sterile, pyrogenfreie PVC-Beutel abgefüllt und etikettiert.

Die etikettierten Beutel werden bei einer Temperatur von <-60°C tiefgefroren, sodass die Temperatur im Inneren der Beutel <-30°C erreicht. Bei dieser Temperatur (≤-30°C) werden die Beutel gelagert.Messung der Bindung von Substanzen an verschiedene Albumin-Präparationen

Eine direkte Methode zur Bestimmung der Bindungseigenschaften von Substanzen an Albumin bietet die Größenausschluss-Chromatographie (SEC, size exclusion chromatography) nach Hummel und Dreyer (Biochim Biophys Acta 1962; 63: 530-532).

Dazu wird eine SEC-Säule mit einer den Bindungsliganden (z.B. Phenylbutazon oder Warfarin) enthaltenden Pufferlösung äquilibriert. Die Absorption im UV-Bereich wird kontinuierlich verfolgt. Das Protein wird auf die Saule aufgetragen und in dem Äquilibrierungspuffer eluiert. Gebundener Ligand eluiert dabei zusammen mit dem Albumin, der ungebundene, meist kleinere Ligand, eluiert entsprechend später. Die Absorption des gebundenen Liganden interferiert dabei meist mit der Absorption des Albumins und möglichen Begleitsubstanzen, wie Stabilisatoren. Der später eluierende 'negative' sog. 'vacancy' Peak wird durch die Depletion des Liganden im nachfolgenden Puffer verursacht, der um so mehr Fläche einnimmt je mehr an das vorher eluierte Albumin gebunden wurde. Koizumi et al. (Biomed Chromatogr 1998; 12: 203-210) verwendeten diese Methode in leicht modifizierter Form zur Untersuchung der Bindungskapazitäten von Substanzen an Albumin bzw. deren Affinitäten, indem beispielsweise konstante Konzentrationen Albumins in separaten Läufen mit steigenden Mengen des Liganden versetzt wurden und so die Bindungskapzität in Form von Albuminzu-Substanz Ratios.ermittelt werden konnten.

Es wurde eine Biosep-SEC-S 4000 Säule, 300x4,6 mm micron (Phenomenenx) auf einer Shimadzu. HPLC Anlage für diese Untersuchungen verwendeten. Die Pufferflussrate betrug 0,35 ml/min, wobei die Säule mit 50 mM Kalium-Phosphat Puffer, pH 7.4, äquilibriert worden war. Die Proteinkonzentration betrug 50 µM, das Injektionsvolumen 80 µl. Phenylbutazone wurde bei 263 nm verfolgt, Warfarin bei 308 nm. Die linearen Absorptionsbereiche waren zuvor bestimmt worden.

Es wurde das in dieser Anmeldung beschriebenc Albumin verwendet (1), sowie zwei kommerziell erhältliche (stabilisierte) Albumin-Präparate (2,3). Es handelte sich dabei um 20%ige Albuminlösungen.

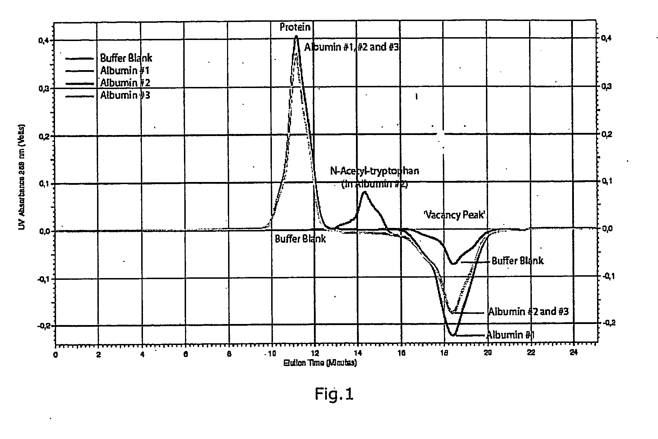

Fig. 1 stellt eine Überlagerung vier verschiedener Chromatogramme dar, wobei die Säule in 50 µM Phenylbutazon äquilibriert war (in Phosphat-Puffer). Bei einer Retentionszeit von 11 Minuten eluierte das Albumin zunächst, wobei der Peak die Summe aus Protein-Absorption und der der gebundenen Substanz anzeigt. Bei 14,5 min zeigt sich im Falle eines kommerziellen Albumin in der Regel ein N-Acetyl-Tryptophan (Stabilisator) Peak. Nach 18,5 min erscheint der 'vacancy' Peak in Form einer 'negativen' Absorptionsdarstellung relativ zum Level des Äquilibrierungspuffer samt Substanz. Je höher (im negativen Sinne) dieser Peak ist bzw. je größer die Peakfläche, um so mehr Substanz hat an das vorher eluierte Albumin gebunden.

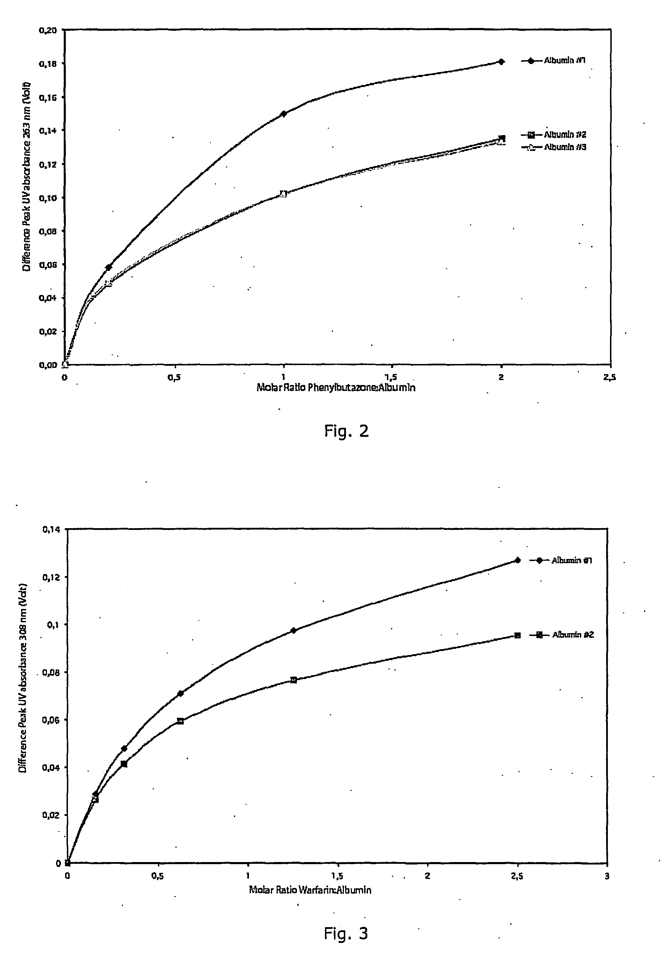

Fig. 2 zeigt die UV-Absorptionen dreier Phenylbutazon-Konzentrationen gebunden an Albumin (nach Subtraktion des Puffer-Peaks). Dazu wurden zwei kommerziell erhältliche (Caprylat und N-Acetyl-Tryptophan enthaltende) Albumine, sowie das nach dem in dieser Anmeldung beschriebenen Verfahren hergestellte Albumin chromatographiert und die Bindungsqualitäten verglichen. Bei vergleichbaren molaren Konzentrationen von Phenylbutazon zu Albumin zeigt sich klar, dass der Peak in Hohe und Fläche eindeutig größer im Falle des neuen Albumins sind. Dies gilt in ähnlicher Weise für das zweite Beispiel, nämlich Warfarin, wie in Fig. 3 dargestellt.

Diese Ergebnisse unterstreichen, dass das kommerzielle Albumin dem hier beschriebenen Albumin in seiner Bindungseigenschaft unterlegen ist.

### Vergleich der Bindungskapazität von RP-18-Säulen im Vergleich zu Polystyrol-Divinylbenzol-Polymeren (Amberchrome 161 M).

Testsystem: Säulenvolumen: 44 ml
Flussrate: 4 ml/min
Die Säule wurde mit einer 1% Triton X-100 Lösung geladen. Der Triton X Gehalt im Eluat nach jedem Säulenvolumen mittels Umkehrphasen HPLC gemessen. Wenn Triton im Eluat nachweisbar war, war die Kapazität des Gels erreicht.

### Ergebnis:

Das RP-18-Gel bindet 140 mg Triton X-100/ml Gel und das Amberchrome-Gel bindet 160 mg Triton X-100/ml Gel.

## Patentansprüche

1. Verfahren zur Herstellung von Albumin, **gekennzeichnet durch** die Kombination folgender Schritte:
(a) Unterziehen einer ersten wässerigen Albuminlösung einer Behandlung zur Virusinaktivierung nach dem SD-Verfahren **durch** Kontaktierung mit SD-Reagenzien bei einer Temperatur unter 45°C,
(b) Entfernen der SD-Reagenzien **durch** Ölextraktion und anschließender hydrophober Wechselwirkungschromatographie zumindest im wesentlichen, wobei zur Chromatographie eine hydrophobe Matrix, insbesondere eine Matrix, an welcher gegebenenfalls hydrophobe Gruppen gebunden sein können verwendet wird, mit der Maßgabe, dass diese Gruppen aliphatische Gruppen mit C > 24 sind, und eine zweite Albuminlösung erhalten wird, welche
(c) gegebenenfalls mit einem oder mehreren Stabilisatoren aus der Gruppe Zucker, Aminosäuren und Zuckeralkohole versetzt wird, mit der Maßgabe, dass als Stabilisator kein Indol-Stabilisator und keine C₆-C₁₀-Fettsäure eingesetzt wird, wonach
(d) die gegebenenfalls mit Stabilisator versetzte, zweite Albuminlösung endkonfektioniert und sterilfiltriert und gegebenenfalls in Endbehältnisse abgefüllt wird.

2. Verfahren nach Anspruch 1, **dadurch gekennzeichnet, dass** die Virusinaktivierung bei einer Temperatur im Bereich von 25 bis 40 °C vorgenommen wird.

3. Verfahren nach einem der Ansprüche 1 und/oder 2, **dadurch gekennzeichnet, dass** die Virusinaktivierung während eines Zeitraumes im Bereich von 4 bis 6 Stunden vorgenommen wird.

4. Verfahren nach einem der Ansprüche 1 bis 3, **dadurch gekennzeichnet, dass** als Stabilisator Glycin, Glutamat, Arginin, oder Lysin bzw. eine Kombination davon eingesetzt wird.

5. Verfahren nach einem der Ansprüche 1 bis 3, **dadurch gekennzeichnet, dass** als Stabilisator Maltose und/oder Sorbitol eingesetzt wird.

6. Verfahren nach einem der Ansprüche 1 bis 5, **dadurch gekennzeichnet, dass** zur Ölextraktion Rizinusöl eingesetzt wird.

7. Verfahren nach einem der Ansprüche 1 bis 6, **dadurch gekennzeichnet, dass** als hydrophobe Matrix ein Polystyrol-Divinylbenzol-Polymer oder ein Polymer auf Methacrylat-Basis verwendet wird.

8. Verfahren nach einem der Ansprüche 1 bis 7, **dadurch gekennzeichnet, dass** verzweigte oder lineare aliphatische Gruppen mit mehr als 24 C-Atomen an die Matrix gebunden sind.

9. Verfahren nach einem der Ansprüche 1 bis 8, **dadurch gekennzeichnet, dass** die Albuminlösung nach Abfüllung in die Endbehältnisse tiefgefroren wird.

10. Verfahren nach einem der Ansprüche 1 bis 9, **dadurch gekennzeichnet, dass** vor oder nach den Schritten (a), (b) oder (c) gegebenenfalls vorhandene Präkallikrein-Aktivator-Aktivität (PKA) in an sich bekannter Weise entfernt wird.

11. Verfahren nach Anspruch 10, **dadurch gekennzeichnet, dass** die Albuminlösung zur Entfernung der gegebenenfalls vorhandenen Präkallikrein-Aktivator-Aktivität
a) mit Aktivkohle in Kontakt gebracht wird, wonach die Aktivkohle aus der Albuminlösung entfernt wird, oder
b) einer Ionenaustauschchromatographie unterzogen wird.

12. Verfahren nach Anspruch 11, **dadurch gekennzeichnet, dass** der Schritt (A) bei einer Albuminkonzentration zwischen 1 und 25 Gew.-% durchgeführt wird.

13. Verfahren nach Anspruch 12, **dadurch gekennzeichnet, dass** die Albuminkonzentration zwischen 5 und 10 Gew.-% beträgt.

14. Verfahren nach Anspruch 11, **dadurch gekennzeichnet, dass** der Schritt (B) bei einer Albuminkonzentration zwischen 5 und 10 Gew.-% durchgeführt wird.

15. Verfahren nach einem der Ansprüche 11 oder 14, **dadurch gekennzeichnet, dass** der Ionenaustauscher ein Anionenaustauscher ist und die Albuminlösung mit Natriumacetat im Bereich von 100 - 150 mmol/l gepuffert ist und der pH im Bereich von 5,0 - 6,0 liegt.

16. Verfahren nach Anspruch 15, **dadurch gekennzeichnet, dass** der pH < 5,5 ist.

17. Verfahren nach einem der Ansprüche 11 oder 14, **dadurch gekennzeichnet, dass** der Ionenaustauscher ein Kationenaustauscher ist und die Albuminlösung mit Natriumacetat im Bereich von 20 - 30 mmol/l gepuffert ist und der pH im Bereich von 4,8 - 6,0 liegt.

18. Verfahren nach Anspruch 17, **dadurch gekennzeichnet, dass** der pH im Bereich von 4,8-5,2 liegt.

## Claims

1. A process for the preparation of albumin, **characterized by** the combination of the following steps:
(a) subjecting a first aqueous albumin solution to a treatment for virus inactivation by the SD method by contacting it with SD reagents at a temperature of below 45 °C;
(b) removing, at least substantially, the SD reagents by oil extraction followed by hydrophobic interaction chromatography, wherein a hydrophobic matrix, especially a matrix to which hydrophobic groups may optionally be bound, is used for said chromatography, with the proviso that said groups are aliphatic groups with more than 24 carbon atoms, to obtain a second albumin solution to which
(c) optionally one or more stabilizers selected from the group of sugars, amino acids and sugar alcohols are added, with the proviso that no indole stabilizer and no C₆-C₁₀ fatty acid is employed as said stabilizer, whereupon
(d) said second albumin solution to which a stabilizer has optionally been added is subjected to final packaging and sterile filtration and optionally filled into final containers.

2. The process according to claim 1, **characterized in that** said virus inactivation is effected at a temperature within a range of from 25 to 40 °C.

3. The process according to any of claims 1 and/or 2, **characterized in that** said virus inactivation is effected during a period of time within a range of from 4 to 6 hours.

4. The process according to any of claims 1 to 3, **characterized in that** glycine, glutamate, arginine or lysine or a combination thereof is employed as said stabilizer.

5. The process according to any of claims 1 to 3, **characterized in that** maltose and/or sorbitol is employed as said stabilizer.

6. The process according to any of claims 1 to 5, **characterized in that** castor oil is employed for oil extraction.

7. The process according to any of claims 1 to 6, **characterized in that** a polystyrene-divinylbenzene polymer or a methacrylate-based polymer is used as said hydrophobic matrix.

8. The process according to any of claims 1 to 7, **characterized in that** branched or linear aliphatic groups with more than 24 carbon atoms are bound to the matrix.

9. The process according to any of claims 1 to 8, **characterized in that** the albumin solution is deep-frozen after being filled into the final containers.

10. The process according to any of claims 1 to 9, **characterized in that** any prekallikrein activator (PKA) activity which may be present before or after steps (a), (b) or (c) is removed in a per se known manner.

11. The process according to claim 10, **characterized in that** said albumin solution is
a) contacted with active charcoal, followed by removing the active charcoal from the albumin solution; or
b) subjected to ion-exchange chromatography;
to remove said prekallikrein activator activity which may be present.

12. The process according to claim 11, **characterized in that** step a) is performed at an albumin concentration of from 1 to 25% by weight.

13. The process according to claim 12, **characterized in that** said albumin concentration is from 5 to 10% by weight.

14. The process according to claim 11, **characterized in that** step b) is performed at an albumin concentration of from 5 to 10% by weight.

15. The process according to either of claims 11 or 14, **characterized in that** said ion-exchanger is an anion exchanger, and the albumin solution is buffered with sodium acetate within a range of from 100 to 150 mmol/l, and the pH is within a range of from 5.0 to 6.0.

16. The process according to claim 15, **characterized in that** the pH is < 5.5.

17. The process according to either of claims 11 or 14, **characterized in that** said ion-exchanger is a cation exchanger, and the albumin solution is buffered with sodium acetate within a range of from 20 to 30 mmol/l, and the pH value is within a range of from 4.8 to 6.0.

18. The process according to claim 17, **characterized in that** the pH is within a range of from 4.8 to 5.2.

## Revendications

1. Procédé de préparation d'albumine, **caractérisé par** la combinaison des étapes suivantes consistant à :
(a) soumettre une première solution aqueuse d'albumine à un traitement d'inactivation virale selon le procédé de traitement par solvant-détergent (SD) par mise en contact avec des réactifs SD à une température inférieure à 45°C,
(b) éliminer, au moins dans une large mesure, les réactifs.SD par extraction d'huile suivie d'une chromatographie d'interaction hydrophobe, dans lequel est utilisée, pour la chromatographie, une matrice hydrophobe, en particulier une matrice à laquelle des groupes hydrophobes peuvent éventuellement être liés, à condition que ces groupes soient des groupes aliphatiques avec C > 24, et une deuxième solution d'albumine est obtenue, laquelle
(c) est éventuellement mélangée avec un ou plusieurs stabilisants du groupe formé par les sucres, les acides aminés et les polyols, à condition de ne pas mettre en oeuvre, en tant que stabilisant, de stabilisant indol ni d'acide gras en C₆ à C₁₀, après quoi
(d) la deuxième solution d'albumine éventuellement mélangée avec un stabilisant est finalisée et soumise à une filtration stérilisante et éventuellement transvasée dans des récipients finaux.

2. Procédé selon la revendication 1, **caractérisé en ce que** l'inactivation virale est réalisée à une température dans la gamme de 25°C à 40°C.

3. Procédé selon l'une quelconque des revendications 1 et/ou 2, **caractérisé en ce que** l'inactivation virale est réalisée en un intervalle de temps de 4 à 6 heures.

4. Procédé selon l'une quelconque des revendications 1 à 3, **caractérisé en ce que** l'on met en oeuvre, en tant que stabilisant, de la glycine, du glutamate, de l'arginine ou de la lysine, ou une combinaison de ceux-ci.

5. Procédé selon l'une quelconque des revendications 1 à 3, **caractérisé en ce que** l'on met en oeuvre, en tant que stabilisant, du maltose et/ou du sorbitol.

6. Procédé selon l'une quelconque des revendications 1 à 5, **caractérisé en ce que** l'on met en oeuvre, pour l'extraction d'huile, de l'huile de ricin.

7. Procédé selon l'une quelconque des revendications 1 à 6, **caractérisé en ce que** l'on met en oeuvre, en tant que matrice hydrophobe, un polymère polystyrène/divinylbenzène ou un polymère à base de méthacrylate.

8. Procédé selon l'une quelconque des revendications 1 à 7, **caractérisé en ce que** des groupes aliphatiques ramifiés ou linéaires contenant plus de 24 atomes de carbone sont liés à la matrice.

9. Procédé selon l'une quelconque des revendications 1 à 8, **caractérisé en ce que** la solution d'albumine, après transvasement dans les récipients finaux, est congelée.

10. Procédé selon l'une quelconque des revendications 1 à 9, **caractérisé en ce que**, avant ou après les étapes (a), (b) ou (c), une activité d'activateur de prékallikréine (PKA) éventuellement présente est éliminée d'une manière connue en soi.

11. Procédé selon la revendication 10, **caractérisé en ce que** la solution d'albumine, pour l'élimination de l'activité d'activateur de prékallikréine éventuellement présente,
a) est mise en contact avec du charbon actif, après quoi le charbon actif est éliminé de la solution d'albumine, ou
b) est soumise à une chromatographie par échange d'ions.

12. Procédé selon la revendication 11, **caractérisé en ce que** l'étape (A) est réalisée à une concentration d'albumine comprise entre 1% et 25% en poids.

13. Procédé selon la revendication 12, **caractérisé en ce que** la concentration d'albumine est comprise entre 5% et 10% en poids.

14. Procédé selon la revendication 11, **caractérisé en ce que** l'étape (B) est réalisée à une concentration d'albumine comprise entre 5% et 10% en poids.

15. Procédé selon l'une quelconque des revendications 11 ou 14, **caractérisé en ce que** l'échangeur d'ions est un échangeur d'anions, la solution d'albumine est tamponnée avec de l'acétate de sodium dans la gamme de 100 à 150 mmol/L et le pH est dans la gamme de 5,0 à 6,0.

16. Procédé selon la revendication 15, **caractérisé en ce que** le pH est inférieur à 5,5.

17. Procédé selon l'une quelconque des revendications 11 ou 14, **caractérisé en ce que** l'échangeur d'ions est un échangeur de cations, la solution d'albumine est tamponnée avec de l'acétate de sodium dans la gamme de 20 à 30 mmol/L et le pH est dans la gamme de 4,8 à 6,0.

18. Procédé selon la revendication 17, **caractérisé en ce que** le pH est dans la gamme de 4,8 à 5,2.
